(19) Europäisches Patentamt
European Patent Office
Office européen des brevets

(11) Veröffentlichungsnummer: **0 051 229**
**B1**

(12) # EUROPÄISCHE PATENTSCHRIFT

(45) Veröffentlichungstag der Patentschrift: **15.02.84**

(51) Int. Cl.[3]: **C 07 C 47/21,** C 07 C 45/00

(21) Anmeldenummer: **81108833.5**

(22) Anmeldetag: **23.10.81**

(54) **Verfahren zur Herstellung von Citral.**

(30) Priorität: **31.10.80 CH 8124/80**

(43) Veröffentlichungstag der Anmeldung: **12.05.82 Patentblatt 82/19**

(45) Bekanntmachung des Hinweises auf die Patenterteilung: **15.02.84 Patentblatt 84/7**

(84) Benannte Vertragsstaaten: **AT BE CH DE FR GB IT LI NL**

(56) Entgegenhaltungen: **CH - A - 223 067**

(73) Patentinhaber: **F. HOFFMANN-LA ROCHE & CO. Aktiengesellschaft, CH-4002 Basel (CH)**

(72) Erfinder: **Breuninger, Manfred Wilhelm, Dr., Habsburgerstrasse 71, D-7800 Freiburg/Brsg. (DE)**

(74) Vertreter: **Lederer, Franz, Dr. et al, Patentanwälte Dr. Franz Lederer Dipl.-Ing. Reiner F. Meyer-Roxlau Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 051 229 B1

ACTORUM AG

# Verfahren zur Herstellung von Citral

Die vorliegende Erfindung betrifft ein neues, verbessertes Verfahren zur Herstellung von Citral.

Das Verfahren ist dadurch gekennzeichnet, dass man eine Verbindung der Formel

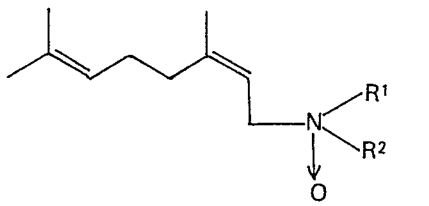

I

worin die Reste $R^1$ und $R^2$ gleich oder verschieden sind und jeweils für $C_{2-6}$-Alkyl stehen, oder $R^1$ und $R^2$ zusammen mit dem N-Atom einen Piperidin-, Pyrrolidin- oder Perhydroazepin-Ring darstellen,
mit einem Anhydrid einer 2 bis 6 Kohlenstoffatome enthaltenden Alkancarbonsäure unter sauren oder basischen Bedingungen umsetzt.

Unter Citral sollen die Isomeren Neral und Geranial als auch Gemische dieser Verbindungen verstanden werden. Als Ausgangsmaterialien der Formel I können die Nerylderivate (Z-Form) oder die Geranylderivate (E-Form) oder Gemische dieser Verbindungen dienen. Das Verhältnis von E/Z hat keinen nennenswerten Einfluss auf Reaktionsablauf oder auf Ausbeute. Diese Ausgangsmaterialien der Formel I sind bekannt oder können nach an sich bekannten Methoden hergestellt werden.

Geeignete Reste $R^1$ und $R^2$ sind Äthyl und Propyl.

Bevorzugt ist Äthyl.

Essigsäure ist die bevorzugte 2 bis 6 Kohlenstoffatome enthaltende Alkancarbonsäure.

Die erfindungsgemässe Umsetzung wird sauer oder basisch durchgeführt, also entweder unter Zuhilfenahme von beispielsweise einer der oben erwähnten Säuren als Lösungsmittel bzw. für den zweiten Fall beispielsweise unter Zugabe von flüssigen tertiären Aminen, z.B. Triäthylamin, Diäthylmethylamin oder Pyridin.

Zweckmässigerweise wird die Verbindung I in einer der oben erwähnten Säure bzw. einem der genannten Amine vorgelegt und das Acylierungsmittel bei Temperaturen von −20° bis 20°C, insbesondere 0 bis 20°C langsam zugegeben. Das molare Verhältnis von I zu Acylierungsmittel beträgt zweckmässigerweise 1:1–5, wobei das Verhältnis 1:3–5 bevorzugt ist.

Hierauf erfolgt, zweckmässigerweise zwischen 0° und 100°C, vorzugsweise zwischen 20° und 60°, die erfindungsgemässe Umsetzung, deren Ablauf z.B. gaschromatographisch verfolgt werden kann.

Zwecks Aufarbeitung werden zum Reaktionsgemisch ein Extraktionsmittel, z.B. ein Äther, als auch Wasser zugegeben. Des weiteren hat sich die Anwesenheit einer Puffersubstanz, wie eines Bicarbonats, und eines Alkohols, z.B. i-Propanol als vorteilhaft erwiesen.

Beispiele

| Edukt | Reaktionsführung | Aufarbeitung | Ausbeute % |
|---|---|---|---|
| (1) | a | A | 51,4–53,7* |
| (1) | b | A | 44,9 |
| (1) | e | D | 40 |
| (2) | c | B | 42,5 |

(1) 1g (4,44 Millimol)

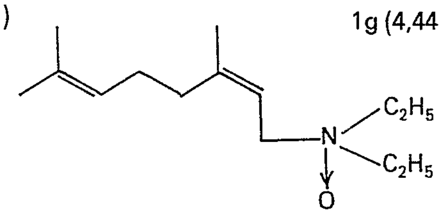

(2) 1g (4,21 Millimol)

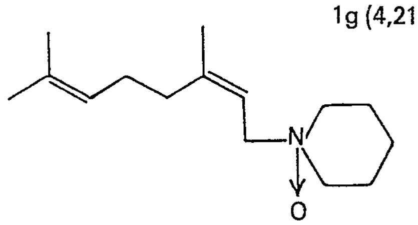

*) 4 Umsetzungen

(a) Lösen des Edukts in 2.0–3.5 g (20–35 Mmol) Triäthylamin; vorsichtige Zugabe von 1.5–2.5 g (15–25 Mmol) Acetanhydrid oder Propionsäureanhydrid bei 0° bis Raumtemperatur unter Rühren; 60 bis 240 Min. Nachrühren bei 40–50°C. Ausbeuten unter Verwendung des Geranylderivates bzw. eines 1:3-Gemisches von Neryl/Geranylderivat analog.

(b) Lösen des Edukts in 1 g (16.7 Mmol) Eisessig; vorsichtige Zugabe von 1.5 g (15 Mmol) Acetanhydrid bei 0° unter Rühren; 240 min. Nachrühren bei 50°.

(c) Wie unter (b), jedoch Zugabe des Anhydrids und Nachrühren bei Raumtemperatur.

(e) Zusatz von 0.13 g (1.5 Mmol) wasserfreiem Natriumacetat als Puffer; rasche Zugabe von 2.5 g (25 Mmol) Acetanhydrid ohne Kühlung, wobei starke Exothermie auftritt; 5 min. Nachrühren.

(A) Zugabe von 0–10 ml Diäthyläther zum Reaktionsgemisch, hierauf von 10 ml Wasser, 5–20 ml i-Propanol, 5–10 g Natriumbicarbonat; 60 min. Rühren bei 40–50°; 3–5malige Extraktion mit Diäthyläther, gaschromatographische Analyse unter Zusatz von β-Cyclocitral als Standard.

(B) wie (A), jedoch ohne Zugabe von i-Propanol.

(D) wie (A), Produktisolation durch Chromatographie des eingeengten Extraktes an Kieselgel unter Verwendung eines Gemisches von Hexan/Äther = 1:1 als Elutionsmittel.

**Patentansprüche**

1. Verfahren zur Herstellung von Citral, dadurch gekennzeichnet, dass man eine Verbindung der Formel

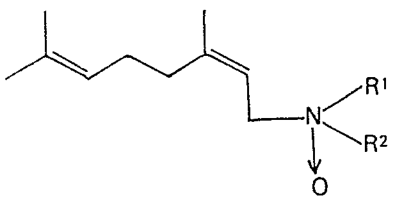

I

worin die Reste $R^1$ und $R^2$ gleich oder verschieden sind und jeweils für $C_{2-6}$-Alkyl stehen, oder $R^1$ und $R^2$ zusammen mit dem N-Atom einen Piperidin-, Pyrrolidin- oder Perhydroazepin-Ring darstellen, mit einem Anhydrid einer 2 bis 6 Kohlenstoffatome enthaltenden Alkancarbonsäure unter sauren oder basischen Bedingungen umsetzt.

2. Verfahren nach Anspruch 1, dadurch gekennzeichnet, dass $R^1$ und $R^2$ Äthyl darstellen.

3. Verfahren nach Anspruch 1 oder 2, dadurch gekennzeichnet, dass man ein Nerylderivat der Formel I als Ausgangsmaterial verwendet.

4. Verfahren nach einem der Ansprüche 1-3, dadurch gekennzeichnet, dass man die Verbindung der Formel I mit Acetanhydrid umsetzt.

5. Verfahren nach einem der Ansprüche 1-4, dadurch gekennzeichnet, dass man das Anhydrid bei -20° bis 20°C zu der Verbindung I zugibt und die Umsetzung bei 0 bis 100°C durchführt.

**Claims**

1. Process for the manufacture of citral, characterized by reacting a compound of the formula

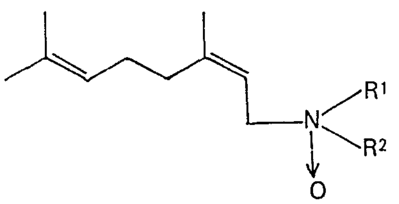

I

wherein the residues $R^1$ and $R^2$ are the same or different and each stand for $C_{2-6}$-alkyl, or $R^1$ and $R^2$ together with the N-atom represent a piperidine, pyrrolidine or perhydroazepine ring, with an anhydride of an alkanecarboxylic acid containing 2 to 6 carbon atoms under acidic or basic conditions.

2. Process according to claim 1, characterized in that $R^1$ and $R^2$ represent ethyl.

3. Process according to claim 1 or 2, characterized in that a neryl derivative of formula I is used as the starting material.

4. Process according to any one of claims 1-3, characterized in that the compound of formula I is reacted with acetic anhydride.

5. Process according to any one of claims 1-4, characterized in that the anhydride is added to the compound I at −20° to 20°C and the reaction is carried out at 0 to 100°C.

**Revendications**

1. Procédé de préparation du citral caractérisé en ce que l'on fait réagir en milieu acide ou basique un composé de formule:

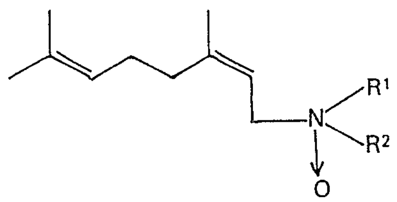

I

dans laquelle les symboles $R^1$ et $R^2$, ayant des significations identiques ou différentes, représentent chacun un groupe alkyle en $C_2$-$C_6$, ou bien $R^1$ et $R^2$ forment ensemble et avec l'atome d'azote un cycle pipéridine, pyrrolidine ou perhydroazépine, avec un anhydride d'un acide alcane-carboxylique contenant 2 à 6 atomes de carbone.

2. Procédé selon la revendication 1, caractérisé en ce que $R^1$ et $R^2$ représentent des groupes éthyle.

3. Procédé selon la revendication 1 ou 2, caractérisé en ce que l'on utilise en tant que produit de départ un dérivé nérylique de formule I.

4. Procédé selon l'une des revendications 1 à 3, caractérisé en ce que l'on fait réagir le composé de formule I avec l'anhydride acétique.

5. Procédé selon l'une des revendications 1 à 4, caractérisé en ce que l'on ajoute l'anhydride à une température de −20°C à 20°C au composé I et on effectue la réaction à une température de 0 à 100°C.